# EUROPEAN PATENT SPECIFICATION

(11) **EP 3 634 160 B1**
(45) Date of publication and mention of the grant of the patent: **28.07.2021**
(21) Application number: 17727901.5
(22) Date of filing: 06.06.2017
(51) Int. Cl.: A24F 1/00, A24F 47/00, A61M 15/06, B65D 47/36, B65D 47/38

(54) **INHALER DEVICE AND CONSUMABLE CARTRIDGE**
INHALATORVORRICHTUNG UND VERBRAUCHSKARTUSCHE
DISPOSITIF INHALATEUR ET CARTOUCHE DE PRODUIT CONSOMMABLE

(43) Date of publication of application: 15.04.2020
(73) Proprietor: JT International SA, 1202 Geneva (CH)
(72) Inventor: HOLROYD, Simon, London Greater London W6 0AU (GB); ROWE, Christopher J., Cambridge Cambridgeshire CB1 7BL (GB); ROGAN, Andrew R. J., Moray Scotland IV36 2HL (GB)
(74) Representative: Gill Jennings & Every LLP
(86) International application number: PCT/EP2017/063739
(87) International publication number: WO 2018/224132

(56) References cited:
- EP-A1- 3 175 722
- WO-A1-2015/165814
- US-A1- 2014 190 477

## Description

### Field of the Invention

The present invention relates to a consumable cartridge for an inhaler device, such as a personal vaporizer device or an electronic cigarette. The present invention also relates to an inhaler device, configured to receive such a consumable cartridge.

### Background of the Invention

Personal inhaler devices, such as electronic cigarettes or "e-cigarettes" as they are also known, have gained in popularity over the past ten years as an alternative to traditional smoking articles, like cigarettes, cigars, and cigarillos. E-cigarettes typically use a vaporizer to vaporize a vaporizing liquid such as a nicotine liquid from an internal reservoir. Many e-cigarette are so called "open tank systems", where the user refills an internal reservoir with a refill bottle. Because the technology employed in personal vaporizer devices is still quite young, developments in the design and configuration of such devices are on-going to improve their performance and their reliability, as well as their ease of production and their production costs.

Some inhaler devices are configured to be recharged with vaporizing liquid in the form of consumable cartridges or so-called "cartomizers" which are consumable items including a reservoir of e-liquid together with a vaporizing unit comprising a wick and a heating element. These consumable cartridges or cartomizers eliminate the need of manual refilling of a reservoir of the e-cigarette and are easy to use by a consumer. However, they are typically not hermetically sealed and so the e-liquid may deteriorate over time if stored for any length of time before being used. Also, cartomizers have the disadvantage that the vaporizing unit (i.e. the heater and the wick) needs to be replaced together with the e-liquid reservoir.

US2014/190477 discloses a replaceable universal atomizing head. WO2015/165814 discloses a container having a heater for an aerosol-generating device, and aerosol-generating device.

### Summary of the Invention

In view of the above, an object of the present invention is to provide a new and improved consumable cartridge for an inhaler device, such as a personal vaporizer device or an electronic cigarette. In particular, it would be desirable to provide such a new and improved consumable cartridge, which ensures a satisfying and reproducible vaping experience.

It would be desirable to provide a consumable cartridge, which reduces the exposure to the e-liquid for the user.

It would also be desirable to provide a consumable cartridge that cannot be refilled.

According to a first aspect, the present invention relates to a consumable cartridge for an inhaler device, the consumable cartridge comprising:
- a casing defining a cavity, the casing comprising a first region and a second region; and
- an elastic bladder located within the cavity of said casing and enclosing a vaporizing liquid;
wherein the first region of the casing comprises a pierceable portion, and the second region comprises a perforated portion; and wherein the bladder is disconnected from the casing in the first region, and wherein the bladder is configured to rupture or to be cut when it is pierced by a piercing member of the inhaler device such that the ruptured or cut area of the bladder creates an opening which is significantly larger than the cross sectional area of the piercing member, wherein the bladder is fixedly connected to the casing at a connection point which is distal from the pierceable portion.

Significantly larger means that liquid is able to flow through in-between the piercing member and the bladder to fill up the outer casing. Preferably, the opening in the bladder provides a distance to piercing member which is larger than 1 mm.

The present invention is based on a realization that an inner bladder can be ruptured with a significant deformation so that it cannot be refilled. Furthermore, the casing protects the elastic bladder from rupturing by accident and thus helps preventing access to the elastic membrane. As the casing after use is provided with openings in both the first region and the second region thereof, refilling of the cavity of the casing will also be difficult.

The perforated region is provided with apertures, i.e. "pre-perforated" before the consumable cartridge is introduced inside the inhaler device. Conversely, the piercing step of the consumable cartridge is effectuated as the consumable cartridge is introduced into the cartridge chamber of the inhaler device.

Preferably, in use, the bladder does not seal around the piercing member once it has been cut or ruptured. Instead there is a space between the bladder and the piercing member so that the vaporizing liquid contained by the bladder readily flows out of the elastic bladder into the cavity of the casing.

According to an exemplary embodiment, the casing extends axially between the first region which is located at one axial end of the casing and the second region which is located at the axially opposite end of the casing, and wherein the casing comprises a main part which includes the first region and a cover which is attached to the main part.

Preferably, the cover is perforated and located at the second region so as to form the pre-perforated portion.

According to claim 1, the bladder is fixedly connected to the casing at a connection point, which is distal from the pierceable region.

The connection point may be located on the main part of the casing adjacent to the cover or is located on the cover. Alternatively, the bladder is attached to the casing between the main part and the cover.

According to an exemplary embodiment, the elasticity of the bladder and the location of the connection point enables the bladder to disconnect from the pierceable member after the bladder has been ruptured or cut.

According to an exemplary embodiment, the pierceable region of the cartridge is a weakening of the casing. Alternatively, the pierceable region of the cartridge is an aperture covered by a sealing membrane or film. Alternatively, the pierceable region has on opening on the outer casing, which provides a direct access for the piercing member to the bladder.

According to an exemplary embodiment, the cover comprises a porous material. A porous material enables the casing to provide small apertures that would let liquid from the cavity pass through, but inhibits dirt from entering into the cavity of the casing.

The cover may be formed from a separate part and sealed to the main part of the casing by heat treatment or adhesive.

According to an exemplary embodiment, the axial cross section of the casing is substantially circular.

According to another exemplary embodiment, the axial cross section of the casing is non-circular. The non-circular shape of the casing creates channels (which may carry vapour) between the cartridge chamber and the consumable cartridge. The non-circular cross-section of the casing may be a regular convex polygon, lobe-shaped, an oval shape or a combination of shapes. The formation of (air) channels between the wall of the casing and the cartridge chamber is particularly advantageous in the embodiment where the inhaler device has the vaporizing chamber located in the power supply unit and thus away from the mouthpiece. The air from the vaporizing chamber thus flows across the capsule in-between the cartridge wall and the consumable cartridge when in use.

Preferably, the volume of the bladder (when full of e-liquid) is smaller than the volume of the casing cavity, such that an air pocket is created inside the consumable cartridge prior to use of the cartridge.

According to a second aspect, the present invention relates to an inhaler device configured to receive a consumable cartridge, the inhaler device comprising a mouthpiece section and a power supply unit, wherein the inhaler device comprises:
- a vaporizing unit comprising a heater and a wick located inside a vaporizing chamber,
- a cartridge chamber configured to receive a consumable cartridge, and
- a piercing member located inside the cartridge chamber, said piercing member having a tip portion and a liquid conveying portion,
wherein the wick is located inside the piercing member and having an exposed liquid conveying portion, which extends outside the tubular piercing member, and wherein the heater is located at the exposed liquid conveying portion and wherein the an abutment portion having an abutment surface closes off the cartridge chamber such that it seals the perforated region of the consumable cartridge.

Preferably, the piercing member is substantially tubular and is open at both ends, the tip portion end being located in the cartridge chamber and the other end opening into the vaporising chamber through a plate or separation wall which separates the cartridge chamber from the vaporizing chamber and on which the piercing member is mounted.

The heater and the exposed liquid conveying portion of the wick within the vaporization chamber together form a vaporization unit. The vaporization unit together with the piercing member and connected structures (e.g. the separation plate or wall and associated outer structures of the device) may form a disposable vaporization assembly which can be disposed of and replaced from time to time when the vaporization unit has reached the end of its useful life. In some embodiments, the vaporization unit may be formed as a disposable mouthpiece section. In other embodiments, the vaporization assembly may be formed as a separate assembly which is operable to be interconnected between a mouthpiece section and a power supply unit.

In some preferred embodiments, the cartridge chamber is located between a vapour outlet in the mouthpiece (through which a user draws vapour when in use) and the vaporizing unit. In such an embodiment, the device preferably includes one or more channels through the separation wall or plate and between the cartridge and the inner wall of the cartridge chamber and through the mouthpiece to the vapour outlet.

According to an exemplary embodiment, the cartridge chamber further comprises a resilient member configured to exert an expelling force on the consumable cartridge from the cartridge such that it is separated from the cartridge chamber when the mouthpiece section and the power supply unit are separated from each other.

### Brief Description of the Drawings

For a more complete understanding of the invention and the advantages thereof, exemplary embodiments of the invention are explained in more detail in the following description with reference to the accompanying drawing figures, in which like reference characters designate like parts and in which:

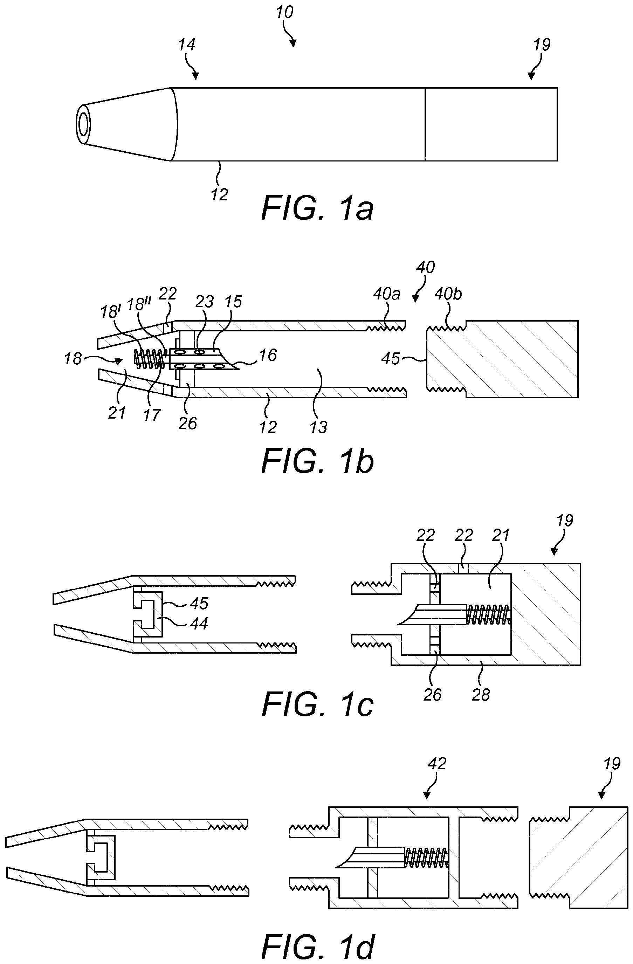

- Fig. 1a: is a schematic perspective view and of the inhaler device according to the present invention;
- Fig. 1b: is a schematic cross-sectional view of an inhaler device according to a first embodiment of the present invention;
- Fig. 1c: is a schematic cross-sectional view of an inhaler device according to a second embodiment of the present invention;
- Fig. 1c: is a schematic cross-sectional view of an inhaler device according to a third embodiment of the present invention;
- Figs. 2(a)-(c): is a series of schematic cross-sectional views showing a consumable cartridge according to one embodiment being deployed in an inhaler cartridge chamber according to an embodiment of the invention;
- Fig. 3(a)-(e): is a series of schematic cross-sectional views of a consumable cartridge according to embodiments of the present invention.
- Fig. 4(a)-(e): are schematic cross-sectional views showing another embodiment of a consumable cartridge being deployed in an inhaler cartridge chamber according to the embodiment of Figs. 2(a)-(c);
- Fig. 5(a)-(e): is a series of schematic cross-sectional views showing a consumable cartridge according to a further embodiment being deployed in an inhaler device according to the present invention;
- Fig. 6 - 8: are schematic cross-sectional perspective views of an inhaler device according to the present invention together with a consumable cartridge of the present invention; and

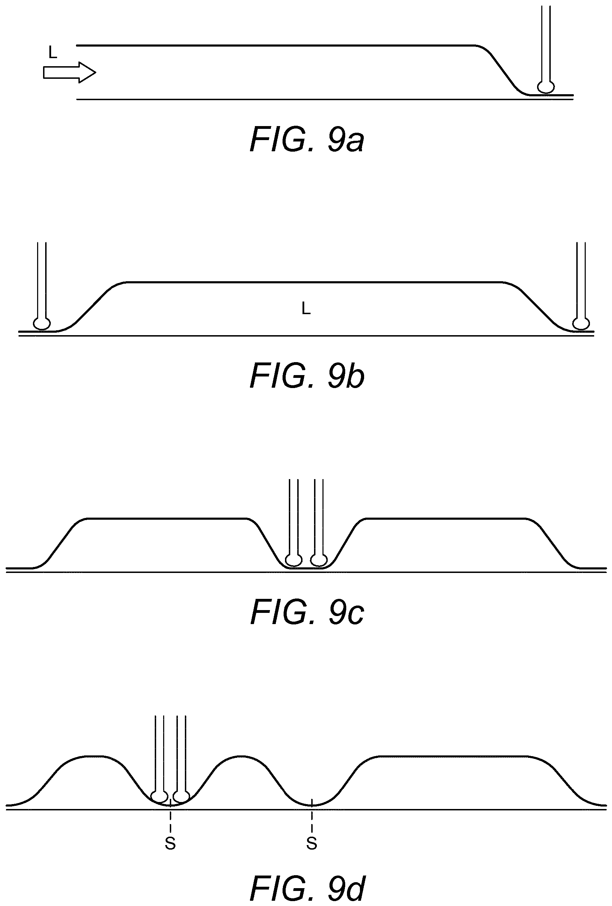

- Figs. 9(a)-(d): illustrate an exemplary manufacturing process of an elastic bladder of the present invention.

The accompanying drawings are included to provide a further understanding of the present invention and are incorporated in and constitute a part of this specification. The drawings illustrate embodiments of the invention by way of example and together with the description serve to explain the principles of the invention. Other embodiments of the invention and many of the attendant advantages of the invention will be readily appreciated as they become better understood with reference to the following detailed description.

It will be appreciated that common and/or well-understood elements that may be useful or necessary in a commercially feasible embodiment are not necessarily depicted in order to facilitate a more abstracted view of the embodiments. The elements of the drawings are not necessarily illustrated to scale relative to each other. It will further be appreciated that certain actions and/or steps in an embodiment of a method may be described or depicted in a particular order of occurrences while those skilled in the art will understand that such specificity with respect to sequence is not actually required. It will also be understood that the terms and expressions used in the present specification have the ordinary meaning as is accorded to such terms and expressions with respect to their corresponding respective areas of inquiry and study, except where specific meanings have otherwise been set forth herein.

### Detailed Description of Embodiments

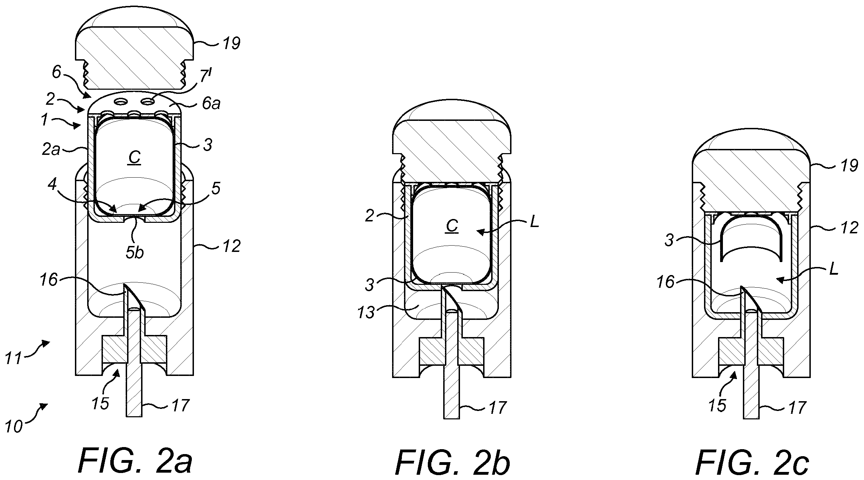

As best seen in figures 1a-1d, an inhaler device 10 according to an embodiment of the present invention is illustrated. The inhaler device 10 comprises a mouthpiece section 14 and a power supply unit 19. The power supply unit 19 is only schematically illustrated, and is in reality typically much larger than shown in the figures to allow for the desired battery volume. The mouthpiece section 14 comprises a housing 12 and an internal cartridge chamber 13. The cartridge chamber 13 is configured to receive consumable cartridges 1 (see e.g. figs. 2a-c) containing a vaporizing liquid *L.*

In the embodiment illustrated in fig. 1b, the mouthpiece section 14 further comprises a vaporizing chamber 21, which houses a vaporizer unit 18. The vaporizer unit 18 comprises a heater 18' and a wick 18".The vaporizing chamber 21 may have air inlet openings 22 for receiving and drawing surrounding air into the vaporizing chamber 21 when a user inhales in the tip of the mouthpiece section 14.

The cartridge chamber 13 comprises a piercing member 15 with an axial extension (aligned with the axial extension of the inhaler device) extending from the cartridge chamber 13 and into the vaporizer chamber 21. The piercing member 15 is a tubular element, which comprises a tip portion 16, configured to pierce or cut the consumable cartridge 1 open. The wick 18" is located inside the piercing member 15 and has an exposed liquid conveying portion 17, which extends outside the tubular piercing member 15 inside the vaporizing chamber 21. The piercing member 15 is preferably provided with lateral holes 23, which communicate with a central channel of the piercing member 15 such that the vaporizing liquid can be absorbed in the wick 18" through capillary action.

The lateral holes 23 provide a mechanism for liquid to soak into the wick along the entire length of the piecing member. The piercing member 15 can be provided with a length that extends along the entire axial inner length of the consumable cartridge 1. The lateral holes 23 can be provided along the entire length of the piercing member 15, whereby a reliable absorption of liquid *L* from the cartridge 1 to the wick can be assured.

The wick 18" comprises fibrous or porous wicking material configured to absorb and diffuse liquid. The wick 18' may for instance comprise natural fibers such as cotton, twined glass or mineral fibers or a porous ceramic material. The piercing member 15 is thus configured to establish a fluid connection between the consumable cartridge 1 and the heater 18' via the wick 18". The liquid *L* flows due to capillary action from the cartridge chamber 13 to the wick 18".

The heater 18 may comprise an electrical resistance heating wire or filament, which is wound around the wick 18". The vaporizer unit 18 and piercing member 15 may be housed in the front tip of the mouthpiece section 14 as illustrated in the embodiment of fig. 1b. As best seen in figs. 1b and 1c, the piercing member 15 may be attached to a bracket 26 or a wall portion separating the cartridge chamber 13 from the vaporizing chamber 21.

In the use of the inhaler device 10, access to the cartridge chamber 13 may be achieved by separating the mouthpiece section 14 from the power supply unit 19, whereby a used consumable cartridge 1 can be discharged from the cartridge chamber 13 and replaced with a new one. The mouthpiece section 14 and the power supply unit 19 can engage with each other by e.g. a threaded connection, a bayonetted coupling, a hinged or a snap-lock connection. The mouthpiece section 14 and the power supply unit 19 may also engage with each other by other types locking mechanism, such as a mechanical arrangement with a lever configured to both lock and release the mouthpiece section 14 and the power supply unit 19 in relation to each other.
Alternatively, as illustrated in figs. 1c and 1d, the vaporizer unit 18 can be located in the housing 19' of the power supply unit 19. An advantage of locating the heater in a housing 28 of the power supply unit 19 is that heater may be located further away from the mouthpiece tip such that less heat is transferred to the user's mouth. In this embodiment, as illustrated in fig. 1c, the vaporizing chamber 21 is located in the housing 28 of the power supply unit 19. The piercing member 15 and the vaporizing unit 18 are fixedly attached to the power supply unit 19, for instance by a connection to a flange 26. The mouthpiece section 14 further comprises an abutment portion 44 having an abutment surface 45 configured to seal the perforated region 6a of the consumable cartridge 1.

In a similar embodiment illustrated in fig. 1d, it is also possible to house the vaporizer unit 18 in a separate vaporization assembly unit 42, which can be disconnected from both the mouthpiece section 14 and the power supply unit 19. The mouthpiece section 14, the cartridge chamber 13 including the vaporizer unit 18 and the power supply unit 19 may be releasably connected to each other in the same way as the mouthpiece section 14 and the power supply unit 19, for instance by a threaded connection. By locating the vaporizer unit in a separate unit, the vaporizer can be replaced independently from the mouthpiece section 14 and the power supply unit 19 if needed.

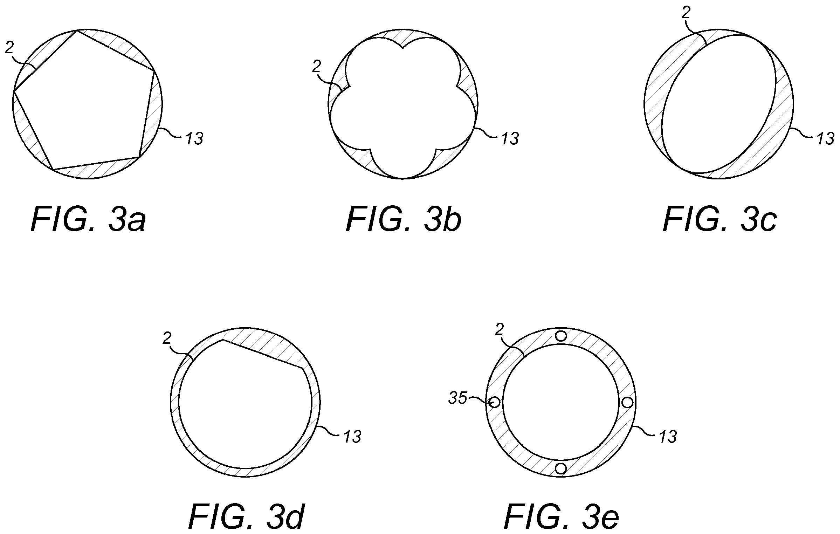

With reference to figs. 2(a)-(c), a consumable cartridge 1 according to the first embodiment of the present invention is further illustrated. The consumable cartridge 1 comprises an outer casing 2 with generally cylindrical cross-section in the axial direction of the consumable cartridge 1. However, the cross-section is not limited to a cylindrical shape, but could be hexagonal, square, oval, multi-lobes etc. as illustrated in figs. 3a-d. Alternatively, the casing 2 of consumable cartridge 1 can be provided with ribs 35. Additionally or alternatively, the cartridge chamber 13 can be provided with a non-circular cross section or ribs. Non-circular cross sections of the consumable cartridge or the cartridge chamber 13 are advantageous when the heater 18' is located in the housing 28 of the power supply unit 30, as the shape can create air channels between the cartridge chamber 13 and the consumable cartridge 1. Furthermore, in this particular embodiment (as illustrated in figs. 1c and 1d), the inlet air flow from air inlets 22 to the vaporizing chamber 21 needs to pass through the cartridge chamber 13, whereby a channelled passage is advantageous.
The consumable cartridge 1 has a first region 4 and a second region 6. A pierceable region 5 and a perforated region 7 are located at the first region 4 and the second end region 6, respectively.

Preferably, the first region 4 and the second region 6 are arranged at opposite distal ends in the axial direction of the consumable cartridge 1. The consumable cartridge includes, at the second end region 6, a perforated cover 6a which is provided, in the present embodiment, with a plurality of apertures 7'. The perforated cover 6a is attached to a main part 2a of the casing 2, and together (i.e. the main part 2a of the casing 2 and the cover 6) form the outer casing 2. The pre-perforated cover 6a may, for instance, be attached to the outer casing 2 by heat treatment or an adhesive. Alternatively, the pre-perforated cover 6a may consist of a porous and permeable cover. A porous cover lets the liquid L pass through the cover whilst minimising the possibility of dirt entering into the cavity *C* of the outer casing 2 prior to it's insertion into the inhaler device 10. The porous cover 6a in the present embodiment is formed from a hydrophobic material, such that its surface remains dry.
The pierceable region 5 may be provided with a pierceable membrane 5b, which in its unpierced state seals the outer casing 2. Optionally, an open seal in the shape of e.g. an O-ring can be arranged at the pierceable opening 5 to provide a snug fit around the piercing member 15. The pierceable membrane 5b inhibits dirt from entering into the cavity C of the outer casing 2. Optionally, the casing 2 can have an opening/aperture allowing direct access for the piercing member 15 to the bladder 3.

An inner elastic bladder 3 is located inside the outer casing 2. The elastic bladder is configured to hermetically enclose and store a vaporizing liquid *L.* The elastic bladder 3 is disconnected from the inner wall of the outer casing 2. Preferably, the elastic bladder 3 is fixedly attached to the main part 2a outer casing 2 at a point *P* adjacent to the pre-perforated cover. The point *P* is preferably located on the pre-perforated cover 6a. Alternatively, the point *P* may be located on the outer casing 2. In the present embodiment, the inner elastic bladder 3 is expanded to more than twice its capacity by the insertion of e-liquid at the time of the manufacture of the consumable cartridgeconsumable cartridge 2 and then is sealed to reduce the rate of degredation of the e-liquid contained within the consumable cartridge prior to use of the consumable cartridge within the inhaler device 10. This is safely achievable using a bladder made of sufficiently elastic material and greatly assists in the rupturing of, and outflow of e-liquid from, the elastic bladder 3.

When the inhaler device 10 is prepared to be used, the mouthpiece section 14 is separated from the power supply unit 19 and a consumable cartridge 1 is inserted into the cartridge chamber 13. As is illustrated in figs. 2a to 2c, during the introduction of the consumable cartridge 1 into the cartridge chamber 13, the piercing member 15 is aligned with the first pierceable region 5 of the consumable cartridge 1.

When the power supply unit 19 is brought into engagement with the mouthpiece section 14, the tip portion 16 of the piercing member 15 is urged against the first pierceable region 5, such that the outer casing 2 is penetrated. The tip portion 16 of the piercing member 15 is then further introduced inside the outer casing 2 and ruptures the elastic bladder 3. As the elastic bladder 3 is ruptured, the vaporizing liquid *L* flows out of elastic bladder and fills up the internal cavity *C* of the outer casing 2. As shown in figure 1c, due to its resilient characteristics, the elastic bladder 3 is retracted towards the point *P* on the perforated cover 6a. This is advantageous, as the elastic bladder 3 moves away from the piercing member 15 and avoids that the elastic bladder 3 covers the lateral holes 23 and the tip portion 16 of the piercing member 15.

Once the consumable cartridge is ruptured, it will no longer hold fluid. At this point the responsibility of containing the fluid falls upon the device in combination with the outer casing 2 of the consumable cartridge 1. This can be alleviated by designing the engagement portion 40 such that it in its initial engagement state doesn't rupture the consumable cartridge. The rupturing step is thus performed once a first connection portion 40a and a second connection 40b portion of the engagement portion 40 are further moved against each other.
The flexible and resilient material enables the elastic bladder 3 to rupture in such a way that the rupture creates an opening in the flexible membrane 3 that is significantly larger than the corresponding cross-sectional area of the tip portion 16 of the piercing member 15. The volume of the internal cavity *C* of the outer casing 2 is larger than the volume of the elastic membrane 3 (in its filled state when it's housing the vaporizing liquid *L).* The volume difference between the internal cavity *C* and the volume taken up by the elastic bladder 3 in its filled state consists of an air volume. This air volume creates an air pocket inside the cartridge chamber 13 when the flexible membrane 3 is ruptured. The air pocket regulates the pressure inside the cartridge chamber 13 and prevents the vaporizing liquid *L* from leaking into the cartridge chamber 13.

When the user wants to start the device, the power supply unit 19 is activated by the user by pressing an activating button (not shown). The electricity from power supply unit 19 activates the heater 18'. By activating the heater 18', the liquid in the wick 18" is heated and vaporized into vapour *V* inside the vaporizing chamber 21. The user can extract the vapour *V* from the device by inhaling in the mouthpiece. As the user inhales, new air enters into the vaporizing chamber 21 through the air inlet opening 22. The air inlet opening 22 can be a single or a plurality of openings formed in housing 12 or mouthpiece 14.

Once the consumable cartridge 1 is depleted, the inhaler device 10 may be opened and the consumable cartridge 1 can be removed. The consumable cartridge 1 maybe manually removed by pulling it out from the cartridge chamber 13. In order to facilitate the removal of the consumable cartridge, a grip, such as a protrusion or a flap can be located on the first end region 4 or the second end region 6 of the outer casing 2 of the consumable cartridge 2. The grip is located on the end region 4, 6 exposed to the user and which is disengaged from the cartridge chamber 13 when the power supply unit 19 is separated from the mouthpiece section 14. Alternatively, the inhaler device 10 may further comprise a consumable cartridge ejection mechanism. In a simple embodiment, a consumable cartridge ejection mechanism may comprise a resilient member such as a coil spring or a leaf spring. It would also be possible to provide the inhaler device 10 with a mechanical cartridge ejection mechanism, which may be activated by a lever.
The depleted consumable cartridge 1 has apertures in both the first end region 4 and the second end region 6. Specifically, one of the regions is pre-perforated and the other region is pierced during the use of the consumable cartridge 1. Hence, the consumable has apertures on opposite sides on the casing 2 when it has been used. These apertures provide a refill protection as the cartridge would leak from a second aperture if a user tries to refill the consumable cartridge in a first aperture.

The outer casing 2 and the elastic bladder 3 are manufactured in two separate steps of a manufacturing process of the consumable cartridge 1. The casing 2 is preferably manufactured by using a thermoplastic material in a molding process. The elastic bladder is preferably manufactured by:
- extruding an elongated member,
- closing an end-portion of the elongated member,
- filling the elongated member with a vaporizing liquid *L* until a predefined inner pressure has been reached,
- applying a pair of clamping members arranged at a distance from each other against the elongated member,
- applying a heat to the clamping members such that two sealed regions are created on the elongated member,
- cutting in-between the the two sealed regions,
- connecting the elastic bladder to a pre-perforated cover, and
- placing the cover with the bladder inside the casing.

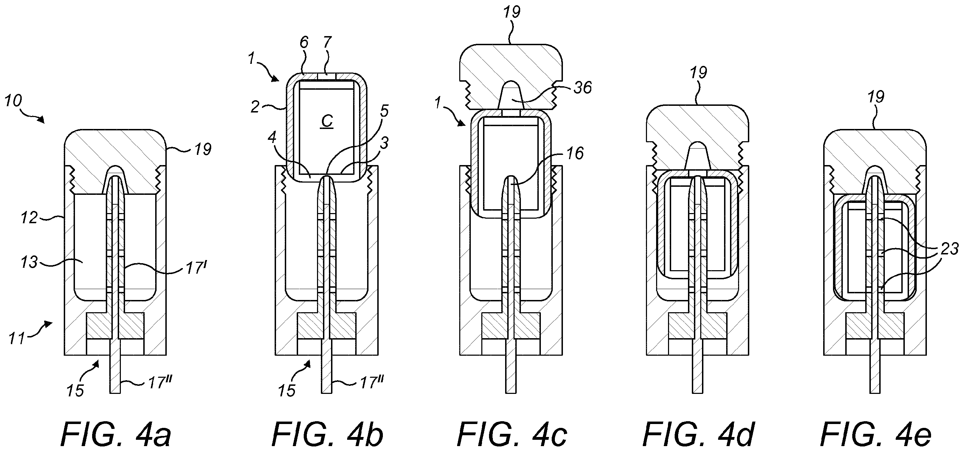

Alternatively, the consumable cartridge can be manufactured by shaping the outer casing 2 in a suitable material, such as thermoplastics. The elastic bladder 3 can be produced in a standardized "balloon shaping process", whereafter the elastic bladders 3 are filled with vaporizing liquid *L* and introduced into the casing 2. The perforated lid 6a is connected to the casing. Preferably, in the same step, a neck of the balloon extends in-between the casing 2 and the perforated cover 6a, such that when the casing 2 and the perforated cover 6a are joined together, the neck of the elastic bladder 3 is connected in a point *P.* Alternatively, the elastic bladder may be connected to the perforated cover 6a in the point *P.*
With reference to figs. 4(a)-(e) of the drawings, a consumable cartridge 1 and inhaler device 10 according to another exemplary embodiment of the present invention is shown. The inhaler device 10 is similar to the first embodiment illustrated in figs. 1a to 1c. However, the inhaler device of figs. 4(a)-(e) differs from the first embodiment in that the piercing member is longer 15, such that it exceeds the axial length of the consumable cartridge 1.

As best seen in figure 4b, the consumable cartridge 1 has a cylindrical casing 2 which encloses a cavity *C* for containing a volume of a vaporizing liquid *L.* The casing 2 is thus directly housing the liquid *L.* The consumable cartridge 1 comprises a first pierceable region 5 and a second pierceable region 7 located on opposite surfaces in the axial direction of the consumable cartridge 1.

As the piercing member 15 is longer than the consumable cartridge in the axial direction, the piercing member is able to pierce through the casing 2 of the consumable cartridge 1 on two distal locations in the axial direction of the consumable cartridge 1. The power supply unit 19 or the end cap may be provided with a recess with 36. The recess 36 creates a space for accommodating the piercing member 15 as it pierces through the consumable cartridge 1 on the second location on the consumable cartridge 1.

The first pierceable region 5 and the second pierceable region 7 may be formed as weakenings in the casing 2. The weakenings create a low resistance zone and facilitates the piercing of the consumable cartridge in those regions. Preferably, the first pierceable region 5 has a weakening and the second pierceable region casing 2 has a cover or a membrane. This would facilitate the filling of the consumable cartridge 1 during manufacturing.

Alternatively, both pierceable regions 5,7 can be formed as apertures in the outer casing 3 and being sealed by the means of a first pierceable membrane 3 and a second pierceable membrane 3'. Preferably, the pierceable regions 5, 7 are centered with respect to the consumable cartridge and the piercing member 15. The sealing membranes 3, 3' may comprise an elastomeric material so that the membranes 3, 3' seal and provide a snug fit around the piercing member 15 when they are pierced through. This prevents a leakage of the liquid L through the membranes 3, 3' and/or through the first pierceable region 5 and the second pierceable region 7.

The first pierceable region 5 and the second pierceable region 7 may be formed as a depression in the wall of the casing 2. A depression protects the weakening from being opened by accident.

In use, as the consumable cartridge 1 is inserted into the cartridge chamber 13, the tip portion 16 of the piercing member 15 is aligned with and passes through the pierceable region 5 of the casing 2, as seen in Fig. 4(b) and Fig. 4(c). As seen in fig. 4(d), when the cartridge 1 is further inserted into the chamber 13, the tip portion 16 of the piercing member 15 is also aligned with the second pierceable region 7.

As seen in fig. 4(e), by rotating or otherwise bringing the power supply unit 19 into connection with the housing 12 of the inhaler device 10, the cartridge 1 is fully driven or forced into the chamber 13. This causes the tip portion 16 of the piercing member 15 to pierce through the second pierceable region 7, such that the piercing member 15 extends through the entire axial length of the casing 2. In the closed inhaler device 10, the the tip portion 16 projects beyond the end region 6 and residing within a corresponding recess in the end cap 19.

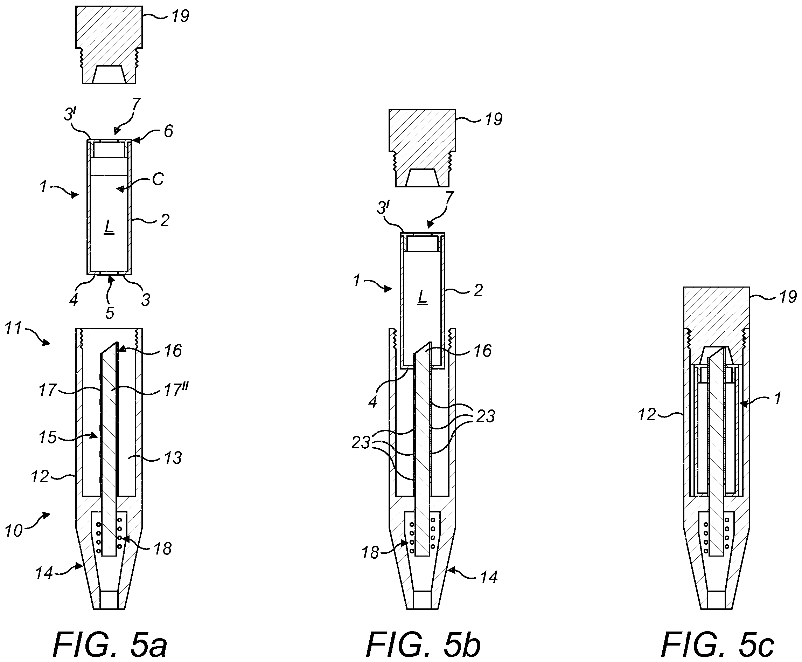

Figs. 5(a)-(c) and figs. 6-8 illustrate another alternative embodiment of a consumable cartridge 1 and an inhaler device 10 of the present invention. The inhaler device 10 is similar to the second embodiment illustrated in figs. 1a to 1c. The consumable cartridge 1 is also similar to the previous embodiment illustrated in figs. 4a-e, but differs in the structure of the pierceable regions 5, 7. The consumable cartridge 1 functions in the same way as the previous embodiment by enabling two pierced apertures in axially opposite locations of the consumable cartridge 1.

The consumable cartridge 1 comprises a generally cylindrical casing 2 which defines a cavity *C* for containing a volume of a vaporizing liquid *L.* The consumable cartridge 1 has a first pierceable region 5 and a second pierceable region 7 both provided with a sheet-like pierceable film or membrane. The outer casing 2 may comprise a main portion 2a and a lid portion 6. An advantage of the lid portion 6 is that it gives a larger access opening for filling the receptacle with vaporizing liquid in a manufacturing process of the consumable cartridge 1.

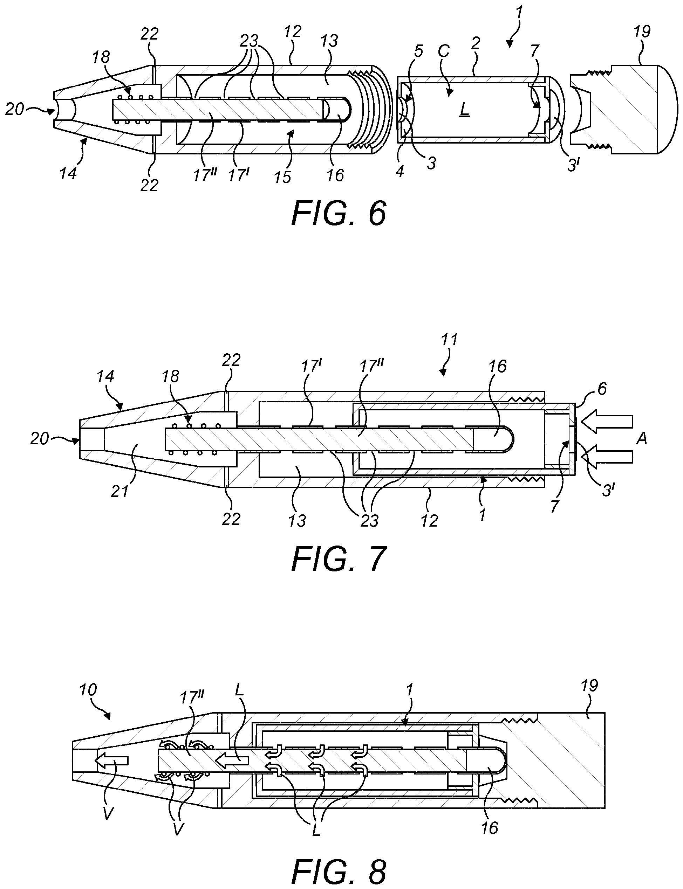

In use, the consumable cartridge 1 is inserted into the cartridge chamber and pierced by the piercing member 15 in the same way as the previous embodiment, whereby the first pierceable region 5 is pierced. By pressing the consumable cartridge 1 in the direction of arrows As seen in fig. 7, the cartridge 1 is further inserted into the chamber 13. Because the piercing member 15 is longer than the casing 2 of the consumable cartridge 1, the tip portion 16 encounters an opposite end region 6 of the casing having a second opening 7 covered and sealed by a membrane 3'. By bringing an end cap 19 (e.g. a power supply unit or battery unit) into connection with the housing 12 of the device 10, the cartridge 1 is fully driven or forced into the chamber 13 and pierced at a second pierceable region 7.

As will be understood by persons of ordinary skill in the art, the power supply unit or battery unit 19 will typically cooperate with a controller or microcontroller module present in the inhaler device 10. These are operatively connected to the aerosol generating means 18 in order to provide electric power or current to the heating coil and to control the vaporization process. A sensor (not shown) may optionally be provided in the piercing member 15 to determine the liquid content remaining in the cartridge 1.

Although specific embodiments of the invention are illustrated and described herein, it will be appreciated by those of ordinary skill in the art that a variety of alternate and/or equivalent implementations exist. It should be appreciated that the exemplary embodiment or exemplary embodiments are examples only and are not intended to limit the scope, applicability, or configuration in any way. Rather, the foregoing summary and detailed description will provide those skilled in the art with a convenient road map for implementing at least one exemplary embodiment, it being understood that various changes may be made in the function and arrangement of elements described in an exemplary embodiment without departing from the scope as set forth in the appended claims. Generally, this application is intended to cover any adaptations or variations of the specific embodiments discussed herein.

It will also be appreciated that in this document the terms "comprise", "comprising", "include", "including", "contain", "containing", "have", "having", and any variations thereof, are intended to be understood in an inclusive (i.e. non-exclusive) sense, such that the process, method, device, apparatus or system described herein is not limited to those features or parts or elements or steps recited but may include other elements, features, parts or steps not expressly listed or inherent to such process, method, article, or apparatus. Furthermore, the terms "a" and "an" used herein are intended to be understood as meaning one or more unless explicitly stated otherwise. Moreover, the terms "first", "second", "third", etc. are used merely as labels, and are not intended to impose numerical requirements on or to establish a certain ranking of importance of their objects.

## Claims

1. A consumable cartridge (1) for an inhaler device (10), the consumable cartridge (1) comprising:
- a casing (2) defining a cavity (*C*), the casing comprising a first region (4) and a second region (6); and
- an elastic bladder located within the cavity of said casing and enclosing a vaporizing liquid (*L*);
wherein the first region (4) of the casing comprises a pierceable portion (5), and the second region comprises a pre-perforated portion (6a); and wherein the bladder is disconnected from the casing in the first region, and wherein the bladder is configured to rupture or to be cut when it is pierced by a piercing member of the inhaler device such that the ruptured or cut area of the bladder creates an opening which is significantly larger than the cross sectional area of the piercing member, wherein the bladder is fixedly connected to the casing at a connection point (*P*) which is distal from the pierceable portion.

2. The consumable cartridge of claim 1 wherein the casing extends axially between the first region which is located at one axial end of the casing and the second region which is located at the axially opposite end of the casing, and wherein the casing comprises a main part (2a) which includes the first region and a cover (6a) which is attached to the main part.

3. The consumable cartridge of claim 2 wherein the cover (6a) is perforated and located at the second region so as to form the pre-perforated portion (6a).

4. The consumable cartridge according to claim 1, wherein the connection point (*P*) is located on the main part of the casing adjacent to the cover (6a) or is located on the cover (6a).

5. The consumable cartridge according to any preceding claim, wherein the bladder is attached to the casing between the main part and the cover (6a).

6. The consumable cartridge according to any preceding claim, wherein the elasticity of the bladder and the location of the connection point (P) enables the bladder to disconnect from the pierceable member after the bladder has been ruptured or cut.

7. The consumable cartridge according to any one of the preceding claims, wherein the pierceable region of the cartridge is a weakening of the casing.

8. The consumable cartridge according to any one of preceding claims1 to 6, wherein the pierceable region of the cartridge is an aperture covered by a sealing membrane or film.

9. The consumable cartridge according to any one of claims 2 to 8, wherein the cover comprises a porous material.

10. The consumable cartridge according to any one of claims 2 to 9, wherein the cover is formed from a separate part and sealed to the main part of the casing by heat treatment or adhesive.

11. The consumable cartridge according to any one of the preceding claims, wherein the axial cross section of the casing is substantially circular.

12. The consumable cartridge according to any one of the preceding claims, wherein the axial cross section of the casing is non-circular.

13. The consumable cartridge according to any one of the preceding claims, wherein the volume of the bladder is smaller than the volume of the casing cavity, such that an air pocket is created inside the consumable cartridge.

14. An inhaler device (10) including a consumable cartridge according to claim 1, the inhaler device comprising a mouthpiece section (14) and a power supply unit (19), wherein the inhaler device comprises:
- a vaporizing unit (18) comprising a heater (18") and a wick (18') located inside a vaporizing chamber (21),
- a cartridge chamber (13) configured to receive a consumable cartridge (1), and
- a piercing member (15) located inside the cartridge chamber (13), said piercing member having a tip portion (16) and a liquid conveying portion (17),
wherein the wick (18') is located inside the piercing member and having an exposed liquid conveying portion (17), which extends outside the tubular piercing member (15), and wherein the heater is located at the exposed liquid conveying portion (17) and wherein the an abutment portion (44) having an abutment surface (45) closes off the cartridge chamber (13) such that it seals the perforated region of the consumable cartridge.

15. The inhaler device (10) according to the preceding claim, wherein the consumable cartridge chamber further comprise a resilient member configured to exert an expelling force on the consumable cartridge from the cartridge chamber such that it is separated from the cartridge chamber when the mouthpiece section and the power supply unit are separated from each other.

## Patentansprüche

1. Verbrauchskartusche (1) für eine Inhalatorvorrichtung (10), wobei die Verbrauchskartusche (1) umfasst:
- ein Gehäuse (2), das einen Hohlraum (C) definiert, wobei das Gehäuse einen ersten Bereich (4) und einen zweiten Bereich (6) umfasst; und
- eine elastische Blase, die sich innerhalb des Hohlraums des Gehäuses befindet und eine zu verdampfende Flüssigkeit (L) einschließt;
wobei der erste Bereich (4) des Gehäuses einen durchstechbaren Abschnitt (5) umfasst, und der zweite Bereich einen vorperforierten Abschnitt (6a) umfasst; und wobei die Blase vom Gehäuse im ersten Bereich getrennt ist, und wobei die Blase ausgelegt ist, derartig zu reißen oder geschnitten zu werden, wenn sie von einem Stechelement der Inhalatorvorrichtung durchstochen wird, dass der zerrissene oder geschnittene Bereich der Blase eine Öffnung schafft, die erheblich größer als die Querschnittsfläche des Stechelements ist, wobei die Blase ortsfest mit dem Gehäuse an einem Verbindungspunkt (P) verbunden ist, der fern vom durchstechbaren Abschnitt ist.

2. Verbrauchskartusche nach Anspruch 1, wobei sich das Gehäuse axial zwischen dem ersten Bereich, der sich an einem axialen Ende des Gehäuses befindet, und dem zweiten Bereich erstreckt, der sich am axial entgegengesetzten Ende des Gehäuses befindet, und wobei das Gehäuse einen Hauptteil (2a) umfasst, der den ersten Bereich und eine Abdeckung (6a) einschließt, die am Hauptteil befestigt ist.

3. Verbrauchskartusche nach Anspruch 2, wobei die Abdeckung (6a) perforiert ist und sich im zweiten Bereich befindet, um den vorperforierten Abschnitt (6a) zu bilden.

4. Verbrauchskartusche nach Anspruch 1, wobei sich der Verbindungspunkt (P) am Hauptteil des Gehäuses angrenzend an die Abdeckung (6a) befindet oder sich an der Abdeckung (6a) befindet.

5. Verbrauchskartusche nach irgendeinem vorhergehenden Anspruch, wobei die Blase zwischen dem Hauptteil und der Abdeckung (6a) am Gehäuse befestigt ist.

6. Verbrauchskartusche nach irgendeinem vorhergehenden Anspruch, wobei die Elastizität der Blase und die Position des Verbindungspunkts (P) der Blase ermöglicht, sich vom durchstechbaren Element zu trennen, nach dem die Blase zerrissen oder geschnitten worden ist.

7. Verbrauchskartusche nach irgendeinem vorhergehenden Anspruch, wobei der durchstechbare Bereich der Kartusche eine Schwächung des Gehäuses ist.

8. Verbrauchskartusche nach irgendeinem der vorhergehenden Ansprüche 1 bis 6, wobei der durchstechbare Bereich der Kartusche eine Öffnung ist, die von einer Dichtungsmembran oder von einem Film bedeckt ist.

9. Verbrauchskartusche nach irgendeinem der vorhergehenden Ansprüche 2 bis 8, wobei die Abdeckung ein poröses Material umfasst.

10. Verbrauchskartusche nach irgendeinem der vorhergehenden Ansprüche 2 bis 9, wobei die Abdeckung aus einem separaten Teil geformt und mittels Wärmebehandlung oder Klebstoff am Hauptteil abgedichtet ist.

11. Verbrauchskartusche nach irgendeinem der vorhergehenden Ansprüche, wobei der axiale Querschnitt des Gehäuses wesentlich kreisförmig ist.

12. Verbrauchskartusche nach irgendeinem der vorhergehenden Ansprüche, wobei der axiale Querschnitt des Gehäuses nicht kreisförmig ist.

13. Verbrauchskartusche nach irgendeinem der vorhergehenden Ansprüche, wobei das Volumen der Blase kleiner als das Volumen des Gehäusehohlraums derartig ist, dass innerhalb der Verbrauchskartusche ein Luftpolster geschaffen wird.

14. Inhalatorvorrichtung (10), die eine Verbrauchskartusche nach Anspruch 1 einschließt, wobei die Inhalatorvorrichtung einen Mundstückabschnitt (14) und eine Stromversorgungseinheit (19) umfasst, wobei die Inhalatorvorrichtung umfasst:
- eine Verdampfungseinheit (18), die ein Heizgerät (18") und einen Docht (18') umfasst, die sich innerhalb einer Verdampfungskammer (21) befinden,
- Kartuschenkammer (13), die ausgelegt ist, eine Verbrauchskartusche (1) aufzunehmen, und
- ein Stechelement (15), das sich innerhalb der Kartuschenkammer (13) befindet, wobei das Stechelement einen Spitzenabschnitt (16) und einen Flüssigkeitstransportabschnitt (17) aufweist,
wobei sich der Docht (18') innerhalb des Stechelements befindet und einen freigelegten Flüssigkeitstransportabschnitt (17) aufweist, der sich außerhalb des rohrförmigen Stechelements (15) befindet, und wobei sich das Heizgerät am freigelegten Flüssigkeitstransportabschnitt (17) befindet und wobei der/ein Anschlagabschnitt (44), der eine Anschlagfläche (45) aufweist, die Kartuschenkammer (13) derartig absperrt, dass er den perforierten Bereich der Verbrauchskartusche abdichtet.

15. Inhalatorvorrichtung (10) nach dem vorhergehenden Anspruch, wobei die Verbrauchskartuschenkammer ferner ein elastisches Element umfasst, das ausgelegt ist, eine ausstoßende Kraft auf die Verbrauchskartusche von der Kartuschenkammer derartig auszuüben, dass sie von der Kartuschenkammer getrennt wird, wenn der Mundstückabschnitt und die Stromversorgungseinheit voneinander getrennt werden.

## Revendications

1. Une cartouche de produit consommable (1) pour un dispositif inhalateur (10) et cette cartouche de produit consommable (1) se compose des éléments suivants :
- un boîtier (2) qui définit une cavité (*C*) et ce boîtier comporte une première zone (4) et une deuxième zone (6), et
- une poche élastique implantée dans la cavité dudit boîtier et contenant un liquide à vaporiser (*L*)
et la première zone (4) du boîtier comporte une partie qui peut être percée (5) alors que la deuxième zone comporte une partie pré-perforée (6a), et la poche est déconnectée du boîtier au niveau de la première zone, et cette poche est conçue pour se rompre ou pour se découper après avoir été percée par un élément de perçage du dispositif inhalateur, de telle sorte que cette rupture ou ce perçage crée une ouverture qui est sensiblement plus importante que la coupe transversale de l'élément de perçage, et cette poche vient se rattacher, de manière fixe au boîtier au niveau d'un point de connexion (*P*) qui se trouve à une extrémité distale de la partie qui peut être percée.

2. La cartouche de produit consommable que décrit la revendication 1, si ce n'est que le boîtier vient s'insérer, sur le plan axial, entre la première zone, qui se situe à une extrémité axiale du boîtier, et la deuxième zone, qui se situe à l'extrémité axiale opposée du boîtier, et si ce n'est que ce boîtier comporte une partie principale (2a) qui englobe la première zone et un couvercle (6a) qui vient se rattacher à la partie principale.

3. La cartouche de produit consommable que décrit la revendication 2, si ce n'est que le couvercle (6a) est perforé et vient se positionner au niveau de la deuxième zone afin de former la partie pré-perforée (6a).

4. La cartouche de produit consommable que décrit la revendication 1, si ce n'est que le point de connexion (*P*) se trouve sur la partie principale du boîtier et est adjacent au couvercle (6a) ou est positionné sur le couvercle (6a).

5. La cartouche de produit consommable que décrit l'une ou l'autre des revendications précédentes, si ce n'est que la poche vient se rattacher au boîtier entre la partie principale et le couvercle (6a).

6. La cartouche de produit consommable que décrit l'une ou l'autre des revendications précédentes, si ce n'est que l'élasticité de la poche et l'emplacement du point de connexion (*P*) permettent de déconnecter la poche de l'élément qui peut être percé après la rupture ou la coupure de cette poche.

7. La cartouche de produit consommable que décrit l'une ou l'autre des revendications précédentes, si ce n'est que la zone qui peut être percée sur la cartouche est un affaiblissement du boîtier.

8. La cartouche de produit consommable que décrit l'une ou l'autre des revendications précédentes 1 à 6, si ce n'est que la zone qui peut être percée sur la cartouche est une ouverture que recouvre une membrane ou un film d'étanchéité.

9. La cartouche de produit consommable que décrit l'une ou l'autre des revendications 2 à 8, si ce n'est que le couvercle se compose d'un matériau poreux.

10. La cartouche de produit consommable que décrit l'une ou l'autre des revendications 2 à 9, si ce n'est que le couvercle est réalisé à partir d'un composant séparé et vient se sceller sur la partie principale du boîtier par traitement thermique ou à l'aide d'un adhésif.

11. La cartouche de produit consommable que décrit l'une ou l'autre des revendications précédentes, si ce n'est que la coupe transversale axiale du boîtier est, essentiellement, circulaire.

12. La cartouche de produit consommable que décrit l'une ou l'autre des revendications précédentes, si ce n'est que la coupe transversale axiale n'est pas circulaire.

13. La cartouche de produit consommable que décrit l'une ou l'autre des revendications précédentes, si ce n'est que le volume de la poche est inférieur au volume de la cavité du boîtier, de façon à ce qu'une poche d'air se crée dans la cartouche de produit consommable.

14. Un dispositif inhalateur (10) composé des éléments suivants :
une cartouche de produit consommable, comme celle que décrit la revendication 1, et cette cartouche de produit consommable comporte une section à embouchure (14) et un bloc d'alimentation électrique (19), et ce dispositif inhalateur se compose des éléments suivants :
- un bloc de vaporisation (18) comportant un élément de chauffage (18") et une mèche (18') qui est implantée dans une chambre de vaporisation (21)
- une chambre à cartouche (13) configurée pour recevoir une cartouche de produit consommable (1), et
- un élément de perçage (15) positionné dans la chambre à cartouche (13) et cet élément de perçage a une pointe (16) et une partie d'acheminement de liquide (17)
et cette mèche (18') est implantée dans l'élément de perçage et a une partie exposée d'acheminement de liquide (17) qui se prolonge à l'extérieur de l'élément tubulaire de perçage (15) et l'élément de chauffage est implanté au niveau de la partie exposée d'acheminement de liquide (17) et la partie en butée (44) comporte une surface de butée (45) qui assure la fermeture de la chambre à cartouche (13) de manière à sceller, de manière hermétique, la zone perforée de la cartouche de produit consommable.

15. Le dispositif inhalateur (10) que décrit la revendication précédente, si ce n'est que la chambre à cartouche de produit consommable comporte, en outre, un élément résilient configuré pour exercer une force d'expulsion sur la cartouche de produit consommable depuis la chambre à cartouche, afin d'en obtenir la séparation par rapport à la chambre à cartouche lorsque la section à embouchure est séparée du bloc d'alimentation électrique.
